# EUROPEAN PATENT APPLICATION

(11) **EP 2 821 787 A1**
(43) Date of publication of application: **07.01.2015**
(21) Application number: 12869695.2
(22) Date of filing: 28.12.2012
(51) Int. Cl.: G01N 33/497, G01N 33/98, G06F 19/00

(54) **BAD BREATH MEASUREMENT SYSTEM**

(30) Priority: 28.02.2012 KR 20120020317
(71) Applicant: Acen Co. Ltd., Gyeonggi-do 443-702 (KR)
(72) Inventor: SONG, Heenam, Yongin-si Gyeonggi-do 449-855 (KR); PARK, Munhee, Hwasung-si Gyeonggi-do 445-717 (KR); LEE, Dongwoo, Osan-si Gyeonggi-do 447-739 (KR)
(74) Representative: Kuhnen & Wacker
(86) International application number: PCT/KR2012/011659
(87) International publication number: WO 2013/129766

(57) **Abstract**

The present invention relates to a system for measuring bad breath or blood alcohol concentration. More specifically, the system comprises: a portable terminal; a bad breath analyzer which has an alcohol sensor and a bad breath sensor mounted therein, and which is connected to an earphone connection terminal of the portable terminal so as to increase compatibility; and a bad breath processing unit which is provided in the portable terminal, and which processes signals measured from the alcohol sensor and the bad breath sensor to output one or more pieces of drinking information and bad breath information on a screen of the portable terminal.

## Description

### TECHNICAL FIELD

The present invention relates to a system for measuring bad breath or blood alcohol concentration, and more specifically, to a bad breath measurement system, in which a bad breath measurement unit is connected to a smart phone to inform a drinking state and a bad breath state of a user through the smart phone, and information on the food taken by the user, information on the bad breath caused by the food, and information on drinking are accumulated so that the user may find out a food inducing the bad breath and duration of a drunken state.

### BACKGROUND ART

Drink-driving causes a big accident since judgment, reflexes and driving views are reduced due to the alcohol accumulated in the body, and, therefore, driving is prohibited by law if alcohol more than a predetermined amount remains in the body after drinking.

Observing drunk drivers, there are a lot of cases in which the drivers drives on their own determination that they did not drink as much as to be caught for drunk driving, and since the amounts of consumed alcohol are entirely determined by their own experiences, it is inaccurate, and, particularly, there will be some arguments with police officers when they are caught for drunk driving.

On the other hand, physiological bad breath occurs due to decreased salivation, an unsanitary mouth or an empty stomach continued for an extended period of time, in addition to food, alcohol, smoking or the like, and there is a problem in that the bad breath may invoke various uncomfortable feelings of other people since it is not self-conscious. In addition, since people do not sense bad breath of their own, there is a problem in that although they actually do not have bad breath, they show a tendency of neurotic depression such as avoiding other people or the like due to their own subjective obsession.

Furthermore, although the bad breath is self-conscious, it is difficult to recognize how bad it is, and thus this may invite a lot of abnormal problems such as avoiding other people in living a social life in a community.

### DISCLOSURE OF INVENTION

### TECHNICAL PROBLEM

Therefore, the present invention has been made in view of the above problems, and it is an object of the present invention to provide a bad breath measurement system, in which a bad breath measurement unit is connected to an earphone connection terminal of a smart phone to extend versatility of the bad breath measurement unit according to the connection terminal and inform a drinking state and a bad breath state of a user through the smart phone, and data according to drinking and bad breath are accumulated so that the user may find out blood alcohol concentration, duration of a drunken state, and a state of the bad breath corresponding to the types of alcohols and foods to cope with the drinking or bad breath states in advance, while exchanging information good for relieving the bad breath or hangovers by sharing and communicating information on the bad breath among users through a communication network.

### TECHNICAL SOLUTION

To accomplish the above object, according to one aspect of the present invention, there is provided a bad breath measurement system including: a portable terminal; a bad breath measurement unit having an alcohol sensor and a bad breath sensor mounted therein and being connected to an earphone connection terminal of the portable terminal so as to increase compatibility; and a bad breath processing unit provided in the portable terminal to process signals measured by the alcohol sensor and the bad breath sensor and output one or more pieces of drinking information and bad breath information on a screen of the portable terminal.

In addition, the bad breath processing unit may further include a drinking analysis unit for comparing the drinking information with drinking data previously set by levels, processing a drinking state by the level, and outputting the processed information on the screen of the portable terminal.

In addition, the bad breath processing unit may further include a bad breath analysis unit for comparing the bad breath information with bad breath data previously set by levels, processing a bad breath state by the level, and outputting the processed information on the screen of the portable terminal.

In addition, the bad breath processing unit may further include a bad breath level setting unit for adjusting criteria of the bad breath data levels through the portable terminal.

In addition, the bad breath processing unit may further include:
an information input unit for inputting information on eaten food and user information according to the drinking information and the bad breath information; a database unit for storing one or more of the data input from the information input unit, the drinking information, the bad breath information and a measurement time; and an information search unit for inputting any one of user and food information through the portable terminal, searching for a data stored in the database unit, and outputting the searched information on the screen of the portable terminal.

In addition, the bad breath processing unit may further include a sound processing unit for informing the information processed by the drinking analysis unit or the bad breath analysis unit as a sound through a speaker of the portable terminal.

In addition, the bad breath measurement unit may further include a sensor selection unit for selectively operating one or more of the alcohol sensor and the bad breath sensor.

In addition, the bad breath measurement unit may use power of the portable terminal.

In addition, the bad breath measurement unit may further include a battery for operating the alcohol sensor and the bad breath sensor inside thereof.

In addition, the bad breath measurement unit may further include a detection unit for detecting a signal of the portable terminal so as to operate only when it is connected to the portable terminal in order to reduce battery consumption.

In addition, the bad breath measurement system may further include a bad breath information sharing unit installed in the portable terminal to perform one or more of sharing the information processed by the bad breath processing unit and performing community activities among users through a wired or wireless communication network.

### ADVANTAGEOUS EFFECTS

In the present invention, since a bad breath measurement unit is inserted into an earphone connection terminal of a smart phone, it can be connected to all kinds of smart phones regardless of a type of a smart phone, and thus compatibility thereof is extremely excellent.

In addition, the present invention is effective in that information on blood alcohol concentration and bad breath according to a bad breath (drinking) state of a user is constructed as a database, and amounts of food or alcohol to be avoided by the user can be controlled by searching for the information.

Furthermore, the present invention is advantageous in that information on the user can be shared through a communication network, and information on relieving hangovers or bad breath can be exchanged through the information sharing.

Furthermore, since the bad breath state can be informed through a sound, even a person of an impaired vision may easily recognize the bad breath state.

### BRIEF DESCRIPTION OF THE DRAWINGS

FIG. 1 is a view schematically showing a bad breath measurement system according to an embodiment of the present invention.
FIG. 2 is a block diagram schematically showing a bad breath measurement system according to an embodiment of the present invention.

### BEST MODE FOR CARRYING OUT THE INVENTION

The preferred embodiments of the present invention will be hereafter described in detail with reference to the accompanying drawings. Furthermore, in the drawings illustrating the embodiments of the present invention, elements having like functions will be denoted by like reference numerals and details thereon will not be repeated.

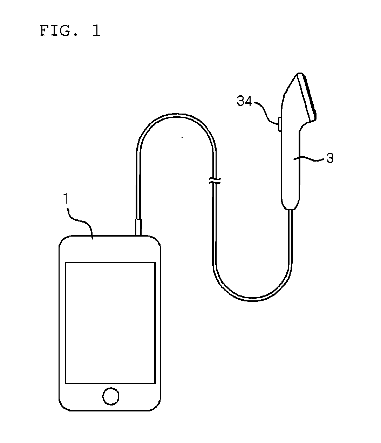

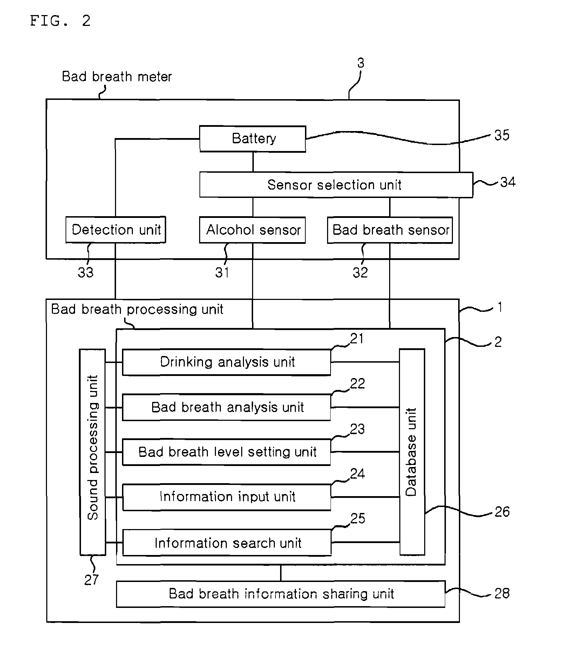

As shown in FIGs. 1 and 2, a bad breath measurement system according to an embodiment of the present invention includes a portable terminal 1, a bad breath measurement unit 3 and a bad breath processing unit 2. In addition, a bad breath information sharing unit 28 is further included.

A general smart phone provided with an earphone connection terminal is used as the portable terminal 1.

The bad breath measurement unit 3 having an appearance formed in the shape of an elliptical bar so as to be easily and roughly grasped includes a sloped inlet (not shown) formed on the top by opening an area to blow in air and an earphone terminal provided at a lower portion, in addition to an alcohol sensor 31, a bad breath sensor 32 and a battery 35 formed inside thereof. In addition, the bad breath measurement unit 3 is connected by inserting the earphone terminal of the bad breath measurement unit 3 into an earphone connection terminal of the portable terminal 1. Here, the bad breath measurement unit 3 may further include a sensor selection unit 34 and a detection unit 33.

The alcohol sensor 31 is installed inside the bad breath measurement unit 3, and a well-known and publicly used alcohol sensor 31 is used to detect alcohol components in the breath exhaled from the mouth through the inlet of the bad breath measurement unit 3. Here, since a technique of detecting the alcohol components by the alcohol sensor 31 is a well-known technique, detailed descriptions thereof will be omitted.

The bad breath sensor 32 is installed inside the bad breath measurement unit 3, and a well-known and publicly used sensor for measuring volatile sulfur compounds is used to detect sulfur compound components in the breath exhaled from the mouth through the inlet of the bad breath measurement unit 3. Here, since a technique of detecting the sulfur compound components by the bad breath sensor 32 is a well-known technique, detailed descriptions thereof will be omitted.

The battery 35 is a general battery, a rechargeable battery or the like generally used and attached to or embedded in the bad breath measurement unit 3 and supplies power to the alcohol sensor 31 and the bad breath sensor 32. For example, when an embedded battery 35 is used, a lithium polymer battery having strong durability, a good recharging efficiency and a long battery life is used. Here, when the battery 35 is embedded in the bad breath measurement unit 3, a recharge terminal (not shown) is installed at a portion of the bad breath measurement unit 3 in order to recharge the battery 35, or a USB conversion connector (not shown) mounted on the earphone terminal may be further included in order to recharge the battery 35 through the earphone terminal of the bad breath measurement unit 3. Accordingly, the battery 35 can be recharged through a USB connection port of a computer (not shown).

The battery 35 described above may be omitted so that the bad breath measurement unit 3 may use power of the portable terminal 1. In this case, if the earphone terminal of the bad breath measurement unit 3 is inserted into the earphone connection terminal of the portable terminal 1, power of the portable terminal 1 is supplied, and the bad breath measurement unit 3 is automatically driven.

The sensor selection unit 34 is installed at a portion of the bad breath measurement unit 3 so that either or both of the alcohol sensor 31 and the bad breath sensor 32 may be selected. Accordingly, since a sobriety test and a bad breath test can be separately or simultaneously processed, power consumption of the battery 35 or the portable terminal 1 can be minimized by selecting a sensor as needed.

The detection unit 33 is installed inside the bad breath measurement unit 3 and detects whether or not the bad breath measurement unit 3 is connected to the portable terminal 1 through the earphone terminal, and the bad breath measurement unit 3 is driven if the bad breath measurement unit 3 is connected, and driving of the bad breath measurement unit 3 is stopped if the connection is released. This is to prevent consumption of the battery 35 by preventing drive of the bad breath measurement unit 3 when connection of the bad breath measurement unit 3 to the portable terminal 1 is released.

The bad breath measurement unit 3 described above may be provided with a separate power button (not shown) in the case where the battery 35 is mounted. At this point, although the bad breath measurement unit 3 is driven using the power button, if the detection unit 33 senses a state of disconnecting the bad breath measurement unit 3 from the portable terminal 1, operation of the bad breath measurement unit 3 is stopped. For example, the power button can be pressed by mistake while the bad breath measurement unit 3 is in a bag, and the battery 35 may continue to be consumed and discharged. Accordingly, the detection unit 33 is provided inside the bad breath measurement unit 3 in order to prevent discharge of the battery 35.

The bad breath processing unit 2 is a unit for processing signals measured by the alcohol sensor 31 and the bad breath sensor 32 and outputting drinking information and bad breath information on the screen of the portable terminal 1, which is an application installed in the portable terminal 1, including a drinking analysis unit 21, a bad breath analysis unit 22, a bad breath level setting unit 23, an information input unit 24, a database unit 26 and an information search unit 25, and a sound processing unit 27 may be further included therein. Here, the bad breath processing unit may be downloaded and installed from a service server (not shown) through a wired or wireless communication network.

The drinking analysis unit 21 receives a signal measured by the alcohol sensor 31, converts the signal into drinking information, and outputs the converted drinking information on the screen of the portable terminal 1. At this point, the drinking analysis unit 21 compares the converted drinking information with drinking data previously set by levels and informs a level of the current drinking state through the screen of the portable terminal 1.

For example, the drinking data previously set by levels is output on the screen of the portable terminal 1 as a message according to a drinking state, such as level one of "Be cautious while driving", level two of "Take a rest", level three of "Don't drive", level four of "Leave the car" or the like. Here, the drinking information may be stored in the database unit 26 according to selection of a user. This will be described below.

The bad breath analysis unit 22 receives a signal measured by the bad breath sensor 32, converts the signal into bad breath information, and outputs the converted bad breath information on the screen of the portable terminal 1. At this point, the bad breath analysis unit 22 compares the converted bad breath information with the bad breath data previously set by levels and informs a level of the current bad breath state through the screen of the portable terminal 1.

For example, the bad breath data previously set by levels is output on the screen of the portable terminal 1 as a message according to a degree of bad breath, such as level one of "No bad breath (Clean)", level two of "Found some bad breath (Kiss with caution)", level three of "Found strong bad breath (Will be slapped if you kiss)", level four of "Found very strong bad breath (Never kiss)" or the like. Here, the bad breath information may be stored in the database unit 26 according to selection of a user. This will be described below.

Here, the user may select either or both of the drinking analysis unit and the bad breath analysis unit to make a measurement.

Since feelings of bad breath previously set in the bad breath data may be different from person to person, the bad breath level setting unit 23 is a component in which the user may change the bad breath data levels through the portable terminal 1. For example, if a degree of bad breath is asked from a counterpart, it may be different from the previously set bad breath data. Accordingly, after asking about a degree of bad breath from a frequently meeting counterpart, the user may directly adjust the bad breath levels so as to take into account the opinion of the counterpart.

The information input unit 24 is a unit for inputting information on a measured person and food corresponding to the bad breath information and may adjust drinking amounts or food to be avoided based on the input information. For example, if a message higher than level three is output on the portable terminal 1 when a user measures bad breath after eating a "Soondae Soup", information on the food is input so that the food eaten by the user may be stored in the database unit 26 described below. At this point, a date and time corresponding to the bad breath information may be automatically input so that duration of the bad breath state may be identified. Accordingly, when a counselling or a seminar is scheduled, it is possible to cope with occurrence of the bad breath by searching for information on the food through the information search unit 25 described below, and thus damage to other persons incurred by the bad breath can be minimized. In addition, when a user drinks "one bottle of Soju and one bottle of beer", information on the alcohols can be input through the information input unit 24, and a drunken state corresponding to the amounts of consumed alcohols may be input by the unit of time. Accordingly, the drunken state corresponding to the amounts of consumed alcohols may be grasped through the information search unit 25 described below, and duration of the drunken state may be grasped.

The database unit 26 is a unit for storing information processed by the drinking analysis unit 21 and the bad breath analysis unit 22 and storing information input from the information input unit 24, i.e., information on the food, user and the like. At this point, a date and time according thereto may be automatically stored in the database unit 26, in addition to the drinking information and the bad breath information. In addition, the database 26 uses the memory embedded in the portable terminal 1. Accordingly, the drinking information, the bad breath information, the food information, the user information, and the date and time can be stored and accumulated to be constructed as a database. Details thereof will be described below.

The information search unit 25 is a unit for searching for information stored in the database unit 26, which mainly searches for food information and user information to grasp drinking amounts and a degree of bad breath of the user. Accordingly, a user may search for information stored in the database unit 26 through the information search unit 25 and grasp and control the drinking amounts and the degree of bad breath.

The information input unit 24 and the information search unit 25 may input information as a voice. Accordingly, even a user who is slow in inputting characters may easily input information using a voice.

The sound processing unit 27 outputs the drinking information and the bad breath information output on the portable terminal 1 and the information searched through the information search unit 25 as a voice through the speaker of the portable terminal 1. That is, a user may be a person in an old aged group or a deadly drunken person having a blurred eye vision, and at this point, the user may not recognize the information output on the portable terminal 1. Accordingly, the sound processing unit 27 may convert the output information into voice information and output the converted voice information through the speaker of the portable terminal 1, and thus the user may grasp the drinking information, the bad breath information and the information searched by the information search unit 25 by hearing the sound.

The bad breath information sharing unit 28 is a unit for sharing information of a user through a wired or wireless communication network. Accordingly, users may share their own information through the bad breath information sharing unit 28 and exchange information useful for hangovers or bad breath through communications. In addition, it may add interest by comparing rankings of the users.

In describing the present invention, although the embodiments are different from each other, the same reference numerals are assigned to the same configurations, and descriptions thereof may be omitted as needed.

While the present invention has been described with reference to the particular illustrative embodiments, it is not to be restricted by the embodiments but only by the appended claims. It is to be appreciated that those skilled in the art can change or modify the embodiments without departing from the scope and spirit of the present invention.

A bad breath measurement system according to the present invention includes a portable terminal; a bad breath measurement unit having an alcohol sensor and a bad breath sensor mounted therein and being connected to an earphone connection terminal of the portable terminal so as to increase compatibility; and a bad breath processing unit provided in the portable terminal to process signals measured by the alcohol sensor and the bad breath sensor and output one or more pieces of drinking information and bad breath information on a screen of the portable terminal.

In addition, the bad breath processing unit may further include a drinking analysis unit for comparing the drinking information with drinking data previously set by levels, processing a drinking state by the level, and outputting the processed information on the screen of the portable terminal.

In addition, the bad breath processing unit may further include a bad breath analysis unit for comparing the bad breath information with bad breath data previously set by levels, processing a bad breath state by the level, and outputting the processed information on the screen of the portable terminal.

In addition, the bad breath processing unit may further include a bad breath level setting unit for adjusting criteria of the bad breath data levels through the portable terminal.

In addition, the bad breath processing unit may further include: an information input unit for inputting information on eaten food and user information according to the drinking information and the bad breath information; a database unit for storing one or more of the data input from the information input unit, the drinking information, the bad breath information and a measurement time; and an information search unit for inputting any one of user and food information through the portable terminal, searching for a data stored in the database unit, and outputting the searched information on the screen of the portable terminal.

In addition, the bad breath processing unit may further include a sound processing unit for informing the information processed by the drinking analysis unit or the bad breath analysis unit as a sound through a speaker of the portable terminal.

In addition, the bad breath measurement unit may further include a sensor selection unit for selectively operating one or more of the alcohol sensor and the bad breath sensor.

In addition, the bad breath measurement unit may use power of the portable terminal.

In addition, the bad breath measurement unit may further include a battery for operating the alcohol sensor and the bad breath sensor inside thereof.

In addition, the bad breath measurement unit may further include a detection unit for detecting a signal of the portable terminal so as to operate only when it is connected to the portable terminal in order to reduce battery consumption.

In addition, the bad breath measurement system may further include a bad breath information sharing unit installed in the portable terminal to perform one or more of sharing the information processed by the bad breath processing unit and performing community activities among users through a wired or wireless communication network.

### INDUSTRIAL APPLICABILITY

The bad breath measurement system according to the present invention can be used for industry in measuring various kinds of smells.

### DESCRIPTION OF SYMBOLS

### (Fig. 2)

1: Portable terminal
2: Bad breath processing unit
21: Drinking analysis unit
22: Bad breath analysis unit
23: Bad breath level setting unit
24: Information input unit
25: Information search unit
26: Database unit
27: Sound processing unit
28: Bad breath information sharing unit
3: Bad breath measurement unit
31: Alcohol sensor
32: Bad breath sensor
33: Detection unit
34: Sensor selection unit
35: Battery

## Claims

1. A bad breath measurement system comprising:
a portable terminal;
a bad breath measurement unit having an alcohol sensor and a bad breath sensor mounted therein and being connected to an earphone connection terminal of the portable terminal so as to increase compatibility; and
a bad breath processing unit provided in the portable terminal to process signals measured by the alcohol sensor and the bad breath sensor and output one or more pieces of drinking information and bad breath information on a screen of the portable terminal.

2. The system according to claim 1, wherein the bad breath processing unit further includes a drinking analysis unit for comparing the drinking information with drinking data previously set by levels, processing a drinking state by the level, and outputting the processed information on the screen of the portable terminal.

3. The system according to claim 1, wherein the bad breath processing unit further includes a bad breath analysis unit for comparing the bad breath information with bad breath data previously set by levels, processing a bad breath state by the level, and outputting the processed information on the screen of the portable terminal.

4. The system according to claim 3, wherein the bad breath processing unit further includes a bad breath level setting unit for adjusting criteria of the bad breath data levels through the portable terminal.

5. The system according to claim 1, wherein the bad breath processing unit further includes:
an information input unit for inputting information on eaten food and user information according to the drinking information and the bad breath information;
a database unit for storing one or more of the data input from the information input unit, the drinking information, the bad breath information and a measurement time; and
an information search unit for inputting any one of user and food information through the portable terminal, searching for a data stored in the database unit, and outputting the searched information on the screen of the portable terminal.

6. The system according to claim 2 or 3, wherein the bad breath processing unit further includes a sound processing unit for informing the information processed by the drinking analysis unit or the bad breath analysis unit as a sound through a speaker of the portable terminal.

7. The system according to claim 1, wherein the bad breath measurement unit further includes a sensor selection unit for selectively operating one or more of the alcohol sensor and the bad breath sensor.

8. The system according to claim 1, wherein the bad breath measurement unit uses power of the portable terminal.

9. The system according to claim 1, wherein the bad breath measurement unit further includes a battery for operating the alcohol sensor and the bad breath sensor inside thereof.

10. The system according to claim 9, wherein the bad breath measurement unit further includes a detection unit for detecting a signal of the portable terminal so as to operate only when it is connected to the portable terminal in order to reduce battery consumption.

11. The system according to claim 1, further including a bad breath information sharing unit installed in the portable terminal to perform one or more of sharing the information processed by the bad breath processing unit and performing community activities among users through a wired or wireless communication network.
